# EUROPEAN PATENT SPECIFICATION

(11) **EP 2 868 844 B1**
(45) Date of publication and mention of the grant of the patent: **26.04.2017**
(21) Application number: 14191114.9
(22) Date of filing: 30.10.2014
(51) Int. Cl.: E04H 15/20, A01G 9/14, A01C 3/02, C12M 1/107, E04B 7/14, E04H 5/08, C12M 1/00

(54) **Structural roofing system and method of using the same**
Strukturelles Dachsystem und Verfahren zu dessen Verwendung
Système structurel de toiture et son procédé d'utilisation

(30) Priority: 30.10.2013 IT MI20131805
(43) Date of publication of application: 06.05.2015
(73) Proprietor: Maco Technology S.r.l., 25050 Provaglio d'Iseo (IT)
(72) Inventor: BECCARELLI, Paolo, I-25050 Provaglio d'Iseo (IT); MAFFEI, Roberto, I-25050 Provaglio d'Iseo (IT)
(74) Representative: Mignini, Davide Giuseppe

(56) References cited:
- WO-A1-2012/136347
- FR-A- 865 372
- FR-A- 1 426 838
- US-A- 4 262 457

## Description

### FIELD OF THE INVENTION

The present invention relates to a structural system used for covering an underneath area, and more specifically, for the covering of various types of structures or surfaces.

It has to be noted that the structural roofing system according to the present invention can be used for the creation of roofing arrangements applied to several typologies of underlying areas and, more specifically, for several typologies of structures used for shelter of people and/or things. This expression is used to indicate that the structural roofing system according to the invention can be used for the covering of structures designated for several uses in the civil and industrial applications which can be employed not only for the shelter of people but also for the protection, the storage, the conservation, etc. of things, such as materials, tools, personal property, liquids, etc.

In detail, the Applicant designed a roofing system according to the invention for the covering of structures in the agricultural field and, more specifically, for the covering of basins for liquids. In detail, the structure can be employed in the field of the production of energy through biomasses obtained from agricultural processes and breeding farms and used for the covering of basins for organic liquids coming from wastes, vegetables, zootechnical liquids, agricultural wastes but also compost produced by digesters for the production of biogas etc. Clearly, this application is provided only as an example and is not limitative, on the contrary, as explained above, the roofing system according to the present invention can be employed for the covering of several structures or areas.

Today there is an increase in the need of structural roofing systems for several typologies of structures or areas where the use of traditional building systems for covers based on reinforced concrete and steel, such as beams, trusses and slabs, are too onerous or even not feasible.

Structural systems based on pneumatic elements are known.

The document US4262457 discloses a structural system self-equilibrated and based on a toroidal pneumatic element connected, and thus constrained, by a set of cables and with an upper membrane and a lower membrane. Through this configuration it is possible to obtain an inner void between the two membranes, which can be used for housing the equipment for the production of energy through solar radiation.

However, the need of a toroidal pneumatic element connected to an upper membrane and a lower membrane in order to obtain the inner void for services, is a considerable limit for the adaptability of the structure disclosed in US4262457.

Another pneumatic structural system is described in the document FR1426838.

A covering is connected to four supporting arches based on pneumatic elements lying directly on the ground. The arches are equipped with a cable which is not able to support from below and keep in a suspended position the pneumatic element.

In addition, the pneumatic elements do not lie on the cable.

In addition, the direct support of the pneumatic arches on the ground and the absence of foundations reduce the efficacious transmission of the forces to the ground and limits considerably the use of the structure described in FR1426838.

Also document FR865372 discloses a pneumatic structural system, used as a hangar, comprising a plurality of arched pneumatic elements lying directly on the ground.

Document WO2012/136347 discloses a textile structural system comprising a plurality of inflatable elements, which can be arranged with a certain curvature standing on the ground for forming a shelter.

Moreover, it is also known the use of flexible covering layers, such as cloths installed above the required area.

However, this solution requires that the flexible covering layer, and more specifically the cloth, should be carefully connected and tensioned in such a way that it does not occupy the area underneath and that it does not allow the ponding of rainwater on the upper surface of the cloth, leading to its downward deformation.

Clearly, with the increase in the size of the area which needs to be covered, the use of flexible covering layers, and more specifically of covering cloths, becomes increasingly difficult and less efficient in terms of protection of the structures underneath and in terms of risk of ponding of rainwater.

This is due to the fact that large cloths are difficult to tension and keep tensioned with the consequent deflection of the central portion of the structure when an adequate support is missing.

At the same time, it is particularly costly to provide a support for the covering layer which requires the construction of a supporting structure realised, for example, by using steel beams or similar solutions which, especially for large spans, involve high manufacturing costs. This aspect becomes more evident when the volume to be covered is exposed to a very corrosive environment, which requires adequate materials characterised by an high resistance to corrosion.

An object of the present invention is to overcome the drawbacks briefly discussed above and to provide a structural roofing system which can be used for several structures and areas and which is economical to be manufactured, rapid to be assembled and, at the same time, resistant and durable over time.

It is also an object of the present invention to provide a structural roofing system which guarantee the correct layout and shape of the covering layer, especially when based on flexible materials, in order to provide the adequate resistance to the environmental loads (wind, water and snow loads) and avoid the accumulation of water, snow or any other material over the covering layer and the consequent downwards deflection which leads to the occupation of the volume underneath. These and other aims are achieved through the structural roofing system according to claim 1 and the method for the construction of roofing arrangements according to claim 12. Additional features and aspects of the system and the method are recited in the respective dependent claims.

The structural roofing system for covering an underneath area, according to the present invention, comprises at least one foundation to transmit the loads of said structural roofing system to the ground, at least one tension element, i.e. an element that, if loaded, works under tension, extending between at least two points of said at least one foundation, at least one pneumatic element supported by said at least one tension element, and at least one covering layer having at least a portion located above said at least one pneumatic element.

More in detail, the at least one pneumatic element keeps at distance at least part of said covering layer from said at least one tension element, when said at least one pneumatic element is at least partially inflated.

In other words, at least part of the covering layer is separated from the at least one tension element by means of the pneumatic element when the latter is at least partially inflated. Advantageously, the pneumatic element allows the control of the distance between the covering layer, placed at least in part above the pneumatic element, and the tension element, which is suspended above the ground due to the connection with at least two points of at least one foundation.

According to an aspect of the invention, the tension element supports at least one pneumatic element in such a way that at least one portion of said pneumatic element lies on said at least one tension element. In this way, the assembly of the roofing system according to the invention is extremely easy, in fact, the process only requires that the pneumatic element is placed above the at least one tension element which allows to support the at least one pneumatic element in a stable and safe way. According to an aspect of the present invention, the tension element is constrained to at least two points of at least one foundation in such a way that it is suspended above the ground and/or above the area underneath covered by the structural system.

In addition, as said above, the pneumatic element lies on the at least one tension element, and the pneumatic element and the covering layer are thus at a distance from the ground and/or from the area underneath the structural system. Thus, the pneumatic element is supported by the tension element at certain distance from the ground and/or the area underneath, preferably in its entirety (i.e. completely).

According to an aspect of the present invention, the pneumatic element is provided with an elongated shape in the direction of extension of said at least one tension element between said at least two points of said at least one foundation. In other words, the pneumatic element has a linear shape which results in a structural element having substantially the shape of a beam.

According to an aspect of the present invention, the pneumatic element has a longitudinal axis extending substantially parallel to the direction of extension of the tension element. In more detail, according to an aspect of the present invention, the pneumatic element has a longitudinal axis extending substantially parallel to the straight line which connects the at least two point of the foundation between which the tension element spans (extends). In other words, according to an aspect of the present invention, the pneumatic element has a rectilinear longitudinal axis.

According to an aspect of the present invention, the pneumatic element has a circular cross section. According to another aspect of the present invention, the pneumatic element is characterised by a substantial cylindrical shape, which can be tapered at the extremities.

In more detail, the elongated shape of the pneumatic element, preferably with a rectilinear longitudinal axis, allows the creation of a structural element with the shape of a beam which confers to the structural roofing system a high level of adaptability and flexibility during the use. In addition, the elongated shape of the pneumatic element allows, through its inflation, the efficacious tensioning of the tension element and of the covering layer arranged above.

On the contrary, in the structural systems described in the documents US4262457 and FR1426838, the inflation of the pneumatic element does not allow the tensioning of the cables and the tensioning of the covering membrane.

In addition, in contrast to the structural systems described in the documents US4262457 and FR1426838, the at least one tension element, the pneumatic element and the covering layer are, according to the invention, different and separated components of the structural roofing system. In fact, the tension element supports the pneumatic element, which lies on the tension element, and supports the covering layer placed above the pneumatic element.

In addition, the presence of the pneumatic element, preferably provided with an elongated shape, allows the inversion of the curvature of the covering layer, preferably when the covering layer is flexible, as compared to the configuration assumed when the covering layer is merely arranged above the at least one tension element. Compared with the known installations, where the flexible covering layer is merely arranged above the area or the structure to be covered, the solution has the advantage of avoiding downwards deflections due to the self-weight and the imposed loads which lead to the accumulation of water or other materials.

Advantageously, the pneumatic element allows to change the shape of the covering layer and creates at least one part of the covering layer with the concavity pointed towards the ground. In this way it is possible to create a simple roofing system which is at the same time extremely resistant and stable.

This result is obtained through the pneumatic element, arranged between the tension element and the covering layer supported by said tension element, which acts as a stabilizer and distributes the forces which are transmitted from the covering layer to the tension element. On the contrary, in the document US4262457, the toroidal pneumatic element is designed to provide an inner void and leads to a hemispheric geometry of the upper membrane characterised by a synclastic curvature.

Advantageously, the covering layer is separated from the ground and suspended in such a way that it does not occupy the area of the structure to be covered. In more detail, the at least one tension element is arranged above the structure in such a way that the pneumatic element and the covering layer, arranged above the pneumatic element, do not occupy the area of the structure underneath.

According to an aspect of the invention, the tension element comprises at least one cable or at least on bar, preferably at least two cables or at least two bars, extending between at least two point of the at least one or more foundations. According to another aspect of the invention, the tension element comprises at least two cables or at least two bars, extending between at least two points of at least one or more foundations.

In addition, it has to be noted that the pneumatic element arranged between the tension element and the covering layer allows to keep suspended the covering layer through the inflation of the body by means of at least one gas. In addition, according to a particularly advantageous aspect of the invention, the presence of the pneumatic element allows to keep at distance the covering layer from the tension element and by controlling the inflation of the pneumatic element it is possible to define the shape of the covering layer which, thus, will be arranged in the desired way, with an inverted curvature and with a tensioned covering layer, in order to resist to the external loads.

According to a preferred embodiment of the invention, the covering layer is manufactured using flexible materials, such as a cloth, and the volume of the pneumatic element can be controlled in order to tension the covering layer, and thus the cloth, obtaining the roofing system supported by the pneumatic element and distanced from the tension element.

In this way, the at least one pneumatic element can change, at least in part, the shape of the flexible covering layer leading to a specific arrangement according to the desired shape and, in more detail, allowing the tensioning of the flexible covering layer.

Advantageously, for example, the at least one pneumatic element can be used for roof ridges, e.g. a raised area on the surface of the structures which allows the discharge of rainwater avoiding water ponding on the covering layer. In other words, the pneumatic element allows to invert the curvature of the flexible covering layer.

It should be noted that the expression "change, at least in part, the shape of the flexible covering layer" is used herein to indicate that the at least one pneumatic element can be inflated in order to tension the flexible covering layer in the desired way occupying a variable volume underneath the covering layer, according to the level of inflation. The modification of the shape of the covering layer does not necessarily require the deformation (strain) of the covering layer but just the tensioning thereof. The covering layer is preferably anchored to a fixed point and/or to the ground and the change of the volume underneath, through the inflation of the pneumatic element, allows the tensioning of the covering layer.

According to an advantageous aspect of the present invention, the covering layer is anchored to a fixed point and/or to the ground through appropriate anchoring means of the structural roofing system according to the present invention. The anchoring means of the covering layer allows the distribution of the forces to the ground, or in correspondence of the fixed point, for example when the covering layer is tensioned through the inflation of the pneumatic element. In fact, the inflation of the pneumatic element leads to the tensioning of the tension element above which the pneumatic element is arranged and the consequent tensioning of the covering layer, arranged above it, which distribute the forces through the anchoring to the ground (or to a fixed point).

Obviously, according to other possible embodiments of the invention, the inflation of the at least one pneumatic element can lead to the deformation of the covering layer and thus to a partial elongation thereof. According to an additional aspect of the present invention, the structural roofing system comprises tensioning means to tension the at least one tension element.

The present invention also relates to the use of a structural roofing system according to the invention for the covering of an area of a structure suitable to accommodate human beings and/or things. In addition, the present invention relates to a structure suitable to accommodate human beings and/or things comprising at least one structural roofing system according to the invention.

In particular, it has to be noted that the structure to be covered can extend at least partially above the ground and/or at least partially below the ground.

The present invention also relates to a method for the construction of roofing arrangements, through at least one structural roofing system according to the invention, comprising the step of providing at least one foundation to transmit loads of said structural roofing system to the ground, the step of constraining at least one tension element between at least two points of said at least one foundation and supporting said at least one pneumatic element by said at least one tension element. In addition, the method comprises the step of placing said covering layer with at least a portion thereof above said at least one pneumatic element and the step of inflating said at least one pneumatic element for spacing said covering layer from said at least one tension element.

The method according to the present invention allows to obtain a considerable simplification of the process of making a covering structure just supporting the covering layer by means of at least one pneumatic element in turn supported by the at least one tension element above which at least a portion of the pneumatic element lies.

It should be noted that the presence of the pneumatic element allows to keep the covering layer, in particular if made with flexible materials, suspended above the covered area without any portion of the element bowed downwards with the consequent limitation of the volume of the area underneath which as to be covered and, thus, it is not suitable for the shelter of human beings and/or things.

As said, the method is particularly advantageous when the covering layer is made of flexible materials, for example made by a cloth. The method according to the present invention allows the efficacious tensioning of the covering cloth in a rapid and simple way through the at least one pneumatic element which is inflated with gas till the covering layer reaches the shape and the level of tension desired.

In particular, according to an aspect of the present invention, the covering layer is anchored to the ground and/or to a fixed point, allowing the tensioning of the covering layer due to the inflation of the pneumatic element supported by the tension element.

Additional aspects and advantages of the present invention will result more clear from the description below, which is exemplary and not limitative, with reference to the attached figures where:
- figure 1 is a perspective view of a possible embodiment of the structural roofing system according to the present invention comprising three pneumatic elements and designed for covering a basin for liquids;
- figure 2 is a perspective view of one pneumatic element supported by at least one tension element and constrained to at least one foundation according to a possible embodiment of the structural roofing system according to the present invention;
- figure 2a is a perspective view of the at least one tension element constrained to at least one foundation according to an additional perspective as compared to the perspective view shown in figure 2, in which the pneumatic element is not represented;
- figure 3 is a lateral view of what shown in figure 2;
- figure 4 is a detailed view of the transversal section A-A represented in figure 3 of a pneumatic element supported by two tension elements according to a possible embodiment of the structural roofing system according to the present invention;
- figure 5 is a lateral view of a possible embodiment of the constraining means of the tension element to said at least one foundation of the structural roofing system according to the present invention.

With reference to the attached figures, a possible embodiment of the structural roofing system 1 according to the present invention will be described.

As mentioned above, the structural roofing system 1 can be employed for covering at least one area underneath with several possible uses. In particular, the area covered by the structural roofing system 1 according to the invention can be part of a structure used to shelter human beings and employed for several applications such as workshops, areas for temporary events, etc. In addition, the structure which can be covered by the structural roofing system 1 can be used to shelter other things. Also in this case the structure can be used for several potential applications such as warehouses for the storage of things.

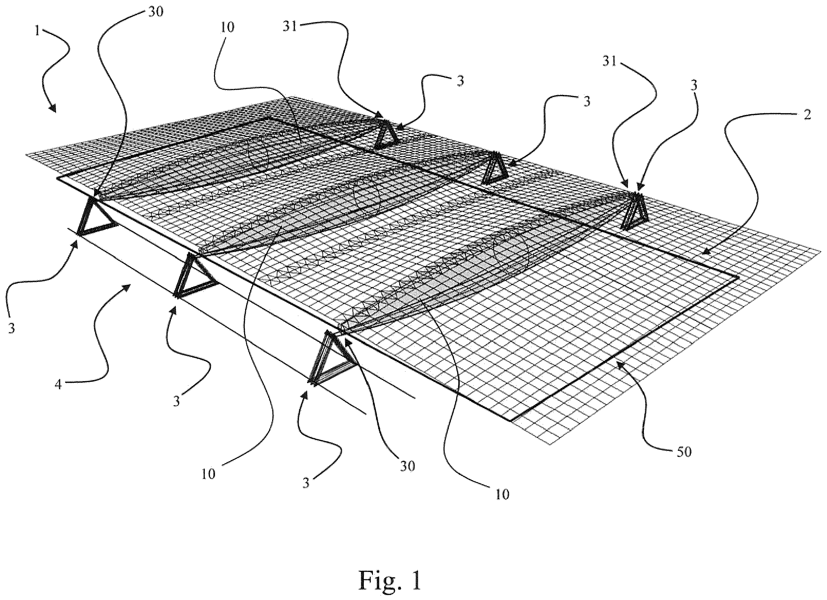

According to a possible embodiment of the invention, shown in figure 1, the structural roofing system can be used for covering an area (structure) underneath employed for the storage of biomasses and, more in detail, for the shelter of a basin 50 extending at least partially below the ground 4 designed for the storage of sewage. The roofing system 1 according to the invention is represented in the perspective view in figure 1 where the structure of the covered underneath area 50, represented in a schematic way, is a basin for the storage of sewage.

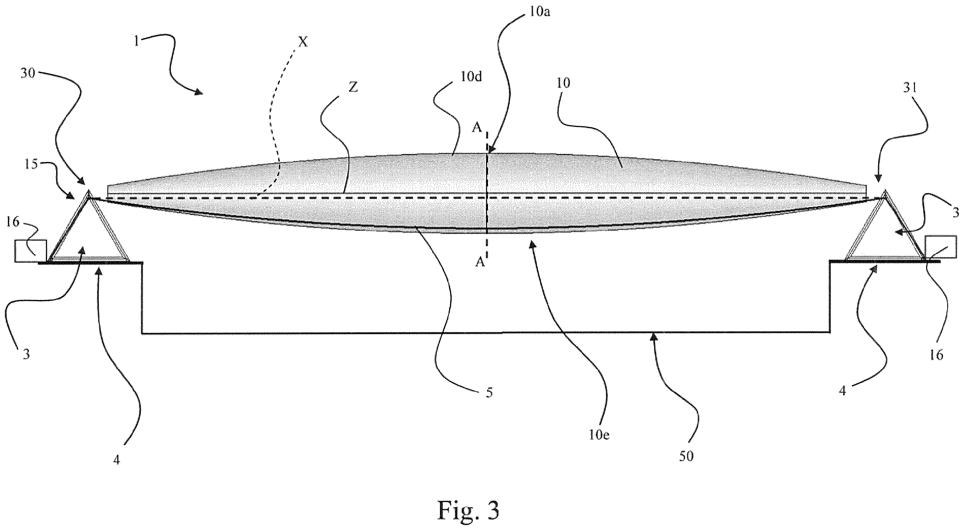

The basin 50 is also visible in the lateral view of figure 3.

Despite the reference to this specific possible use, this application should not be assumed as a limitation of the possible uses but only an example and further applications of the structural roofing systems according to the invention can be used without departing from the scope of the present invention.

The structural roofing system 1, as represented in the attached figures, comprises at least one foundation 3 to transmit the loads applied to the structural roofing system 1 to the ground 4, at least one tension element 5 extending between at least two points 30, 31 of said at least one foundation 3, at least one pneumatic element 10 supported by said at least one tension element 5. As described in detail below, at least one covering layer 2 having at least a portion located above said at least one pneumatic element 10 is supported by said at least one pneumatic element. It should be noted that the covering layer 2 is shown only in the perspective view shown in figure 1 only for reasons of representation. In addition, in figure 1, the covering layer 2 is represented with a mesh in order to allow the correct visualisation of the components of the structural roofing system 1 arranged underneath. Despite this representation, the covering layer 2 is preferably made of a continuous surface without openings.

The term foundation is here used to identify a building element able to transmit the loads applied to the structure connected to said foundation and the other components of the structural roofing system 1, to the ground 4.

Obviously, the foundations used in the structural roofing system 1 according to the invention can be based on different typologies currently in use and can comprise beams, bond-beams, piles and other anchoring systems made with reinforced concrete, steel or other materials.

The foundation used can have a different level of penetration into the ground 4 or can lie on the surface of the ground and comprise, for example, a block like in the example represented in the figures attached. In any case, the at least one foundation of the structural roofing system 1 are designed in order to support the forces transmitted by the roofing structures in such a way that it is completely immovable according to the terminology applied in the building field.

In the attached figures the foundations 3 are represented in a schematic way as rigid blocks (plinth) having a substantially triangular shape which is advantageously used because it provides an efficacious transmission of the forces to the ground being at the same time substantially immovable.

Obviously, other shapes of the blocks 3 can be selected without fall outside the scope of the present invention.

Said blocks can be made with reinforced concrete or other materials known in the building industry. Even though in the attached figures the blocks are arranged on the ground 4, as said above, according to further possible embodiments of the invention the at least one foundation 3 can extend below the ground 4.

It has to be noted that the said foundations can be the same foundations used for the structure 50, or the area, underneath which has to be covered with the structural roofing system 1 according to the invention. On the other hand, the foundations can be just connected or can be made integral part of the foundations of the structure which has to be covered with the system 1.

In particular, according to a preferred embodiment of the invention, the at least on tension element 5 extends between two foundations 3. Having regard to said at least one tension element 5 of the structural roofing system 1, it is designed to act under tension in response to the forces transmitted by the pneumatic element 10, arranged above said tension element. In other words, as described in detail below, the at least on tension element 5 of the structural roofing system 1 supports the at least one pneumatic element 10 having at least a portion lying on said at least one tension element, and the covering layer 2 arranged above the pneumatic element 10. The tension element 5 is tensioned due to the force transmitted by the pneumatic element 10 and transmits the forces to the at least one foundation 3, the tension element being constrained to at least two points of said at least foundation.

More in detail, the tension element 5 is constrained to at least two points 30, 31 of at least one foundation 3 and the tension element is extending between said two points. Advantageously, the tension element is constrained between the at least two points 30, 31 of at least one foundation in such a way that the said tension element is suspended, or at least lifted, above the ground 4 and, more specifically, above the structure 50 which has to be covered. In this way, as described in more detail below, it is possible to support the at least one pneumatic element 10, which in turn supports the covering layer 2, in such a way that the said at least one pneumatic element 10 and the covering layer 2 result at a distance from the ground 4.

Even if in the embodiment of the invention shown in the attached figures the system comprises two distinct foundations 3, between which the tension element 5 spans (extends), according to a possible embodiment of the invention the tension element 5 can span between at least two points 30, 31 of the same foundation 3.

As for example represented in the figures, at least one tension element 5 extends preferably between two points 30, 31 of at least one foundation 3 preferably through the shortest path between the said two points. In more detail, according to a preferred aspect the tension element is extending between the two points 30, 31 of at least one foundation following a straight line X between the two points 30, 31 (see the example in figure 3). Obviously, the tension element extending between the two points 30, 31 of the foundation following the straight line X connecting the two points 30, 31 (direction of the extension) can be curved, for example, downward (see for example the cables represented in figure 3 extending according to the straight line X connecting the points 30, 31 which are, however, curved downwards as compared to the straight line X connecting the points 30, 31).

As previously mentioned, the at least one tension element 5 can have several shapes in order to support the pneumatic element 10. Preferably, the tension element 5 is manufactured with a metallic material, or any other material with an adequate resistance under tension, such as the carbon fibre.

The said at least one tension element 5 comprises at least one cable or at least one bar, preferably at least two cables or at least two bars, extending as said between at least two points 30, 31 of the foundation 3.

It should be noted that the tension element 5 generally comprises an elongated body, preferably with a reduced cross section, or with two smaller dimensions as compared to the extension in length. The term bar is used to describe an elongated body that, in contrast with a cable, is not possible to roll-up. The reinforcing steel bars with a circular cross section are an example of bars usable as tension elements for the structural covering system according to the invention. Clearly, the shape of the cross section is not limited to the circular shape, such as the circular reinforcing steel bars, and bars with a large variety of cross sections can be used.

It should be noticed that the tension element 5, and in particularly cables and bars, can have solid or hollow sections, according to different possible ways of manufacturing.

The use of the cable 5, preferably metallic, for a tension element has the advantage that, by folding the cable, it is possible to reduce the volume of the component during the transport. It should be noted that the cable can be manufactured by using a single wire or by combining more wires in a strand.

In any case, the cross section size and shape, and the material of cables 5 and bars is designed in order to resist to the forces to be transferred to the foundations. In particular, cables and bars should resist to the weight of the pneumatic element supported by the tension element, the covering layer 2 placed at least partially above the pneumatic element 10, and possible static and dynamic loads such as snow and wind estimated during the design.

Clearly, cables and bars 5 can also be combined and used together in order to obtain the tension element.

Moreover, it should be noted that cables and/or bars can be manufactured in a single piece or can comprise multiple pieces connected each other in order to achieve the required length to span between the two points 30, 31 of at least one foundation 3. Adequate elements, known in the technical field but not represented in the attached figures, can be used to connect the parts of said tension elements in order to reach the desired length.

According to a preferred embodiment of the invention, and as represented in the attached figures, the system comprises at least two cables 5, or at least two bars, preferably extending in a parallel way between at least two points 30, 31 of the at least one foundation 3. In particularly, in the embodiment of the invention represented there is a pair of cables 5 extending in a parallel way and suitable to support the pneumatic element 10 arranged above said pair of cables. As described in detail below, with reference to the pneumatic element 10, the said pneumatic element comprises at least a surface 10e oriented towards (facing) the ground and the tension element 5 is arranged between the surface 10e of the pneumatic element oriented towards the ground 4 and the said ground 4, in such a way that it is able to support the pneumatic element.

In the embodiment of the invention shown in the figures, the pneumatic element 10 has a substantially cylindrical shape tapered at its extremities. According to an aspect of the invention, the pneumatic element 10 has a circular cross section. The tension element 5, and in particular the at least two tension elements 5 in the form of cables or bars are preferably arranged in correspondence of the surface 10e oriented towards the ground 4, preferably in the portion of the circumference placed below the horizontal diameter 10b of the circular cross section.

In this way, the pneumatic element 10 is supported from below and its inflation allows the arrangement at a distance of at least the upper portion of the surface 10d, from the tension elements 5 and thus from the ground 4. In this way, the covering layer 2, having at least a portion placed in correspondence of the upper portion 10d of the pneumatic element, can be arranged at a distance from the at least one tension element 5.

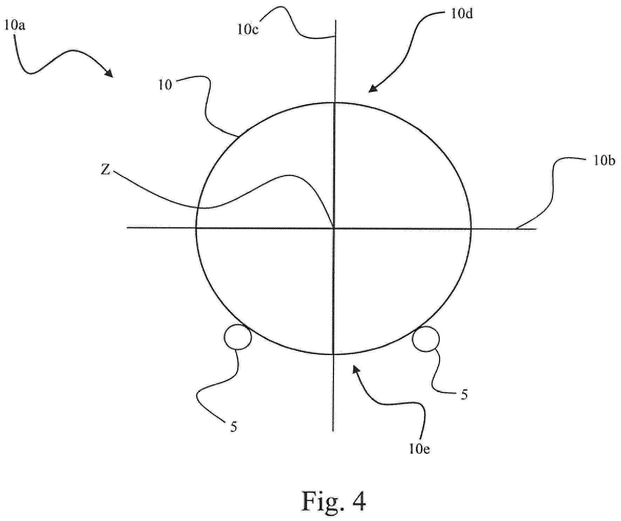

In the embodiment of the invention represented in the figures, where the tension element comprises two cables 5 parallel to each other, said cables are substantially arranged in a symmetric way with respect to the vertical axis 10c, which is preferably the symmetric axis of the pneumatic element considered in the transversal section, see the section view represented in figure 4.

According to other possible embodiments of the invention not represented in the attached figures, the at least one tension element can be made of a woven or knitted net extending between at least two points 30, 31 of the at least one foundation. In alternative, a set of cables parallel each other can be used in order to support one or more pneumatic elements 10.

In addition, it should be noted that the tension element 5 can be connected directly or indirectly to the foundation 3. In the latter case, there could be at least one connection element 3a arranged in between the foundation 3 and the tension element 5, where said connection element allows the connection between the said two components 3 and 5.

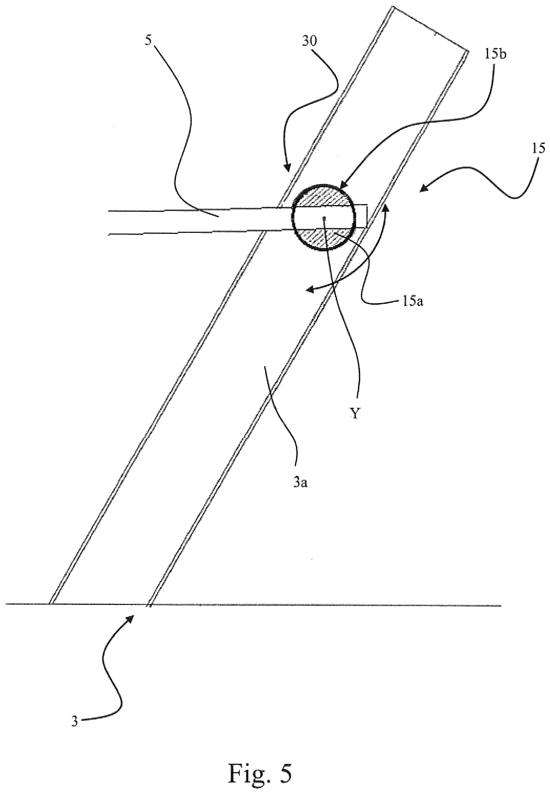

In the lateral view represented in figure 5, which describes a possible connection between the tension element 5 and the foundation 3, there are three connection elements 3a projecting above the foundation 3 even though the lateral view shows only one of said three elements to which the at least one tension element 5 is connected. In detail, three connection elements 3a, in the form of beams, are arranged aligned to each other and shifted in order to provide the space between two of them for one of the two tension elements 5.

According to a possible embodiment of the invention, the structural roofing system 1 according to the invention comprises constraining means 15 which allow the rotation of the tension element 5 around the foundation and/or the connection element 3a which can be used in order to connect the tension element to the foundation 3.

In detail, the constraining means 15 able to rotate around one axis, allow the adjustment of the tension element according to the forces transmitted by the pneumatic element avoiding the anomalous concentration of unwanted stresses and the creation of unwanted shear and bending forces in correspondence of connection between the tension element and the foundation 3.

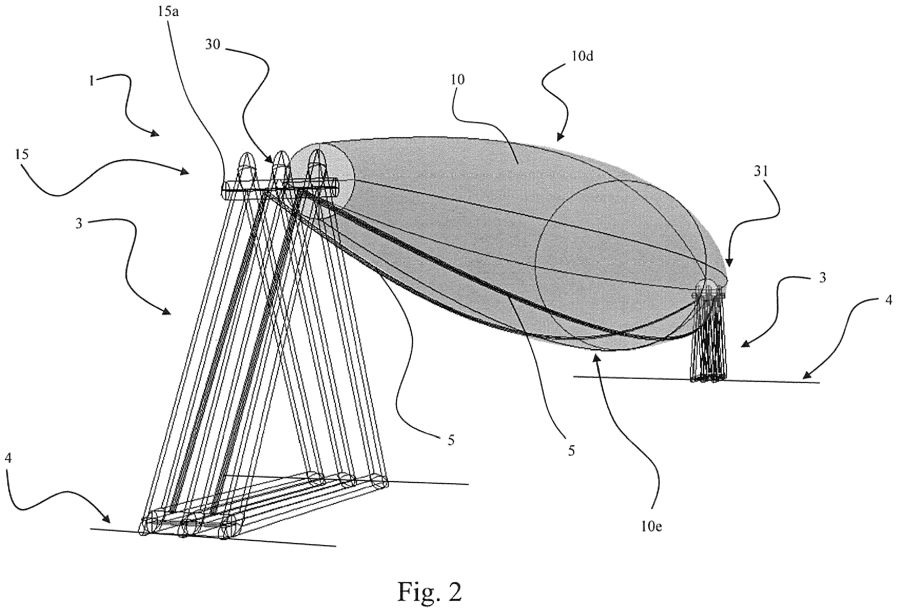

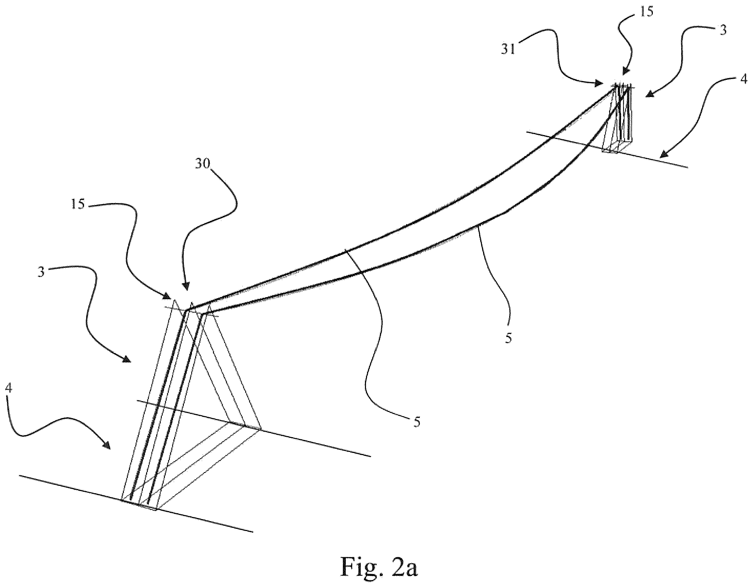

According to a possible embodiment of the invention, and as for example represented in figure 5, the constraining means 15 which allow the rotation of the tension element comprises at leas a pin 15a to which the said tension element, and in particular the two tensioning cables 5, is constrained. The pin 15a is in turn installed in at least one housing 15b in which the said pin can rotate within a certain range of angles. It should be noticed that the constraining means 15, and in particular the pin 15a, are used also in the embodiment of the invention described in the figures 1 - 4, where the said constraining means allow the connection directly to the foundation 3.

It should be noted that the at least one housing 15b in which the pin is installed can be advantageously realised directly in the foundation 3 or in correspondence of the connection element 3a which, as said above, allows the direct connection of the tension element 5 to the foundation 3.

Preferably, the constraining means 15 allow the rotation of the tension element around an axis Y that is substantially orthogonal to the direction X along which the tension element 5 is extending between the points 30, 31 of the foundation.

In the embodiment of the invention represented in the figures, the pin 15a is installed in the housing 15b in such a way that the said pin is aligned to the axis Y, which is substantially orthogonal to the direction X of extension of the tension element 5.

Clearly, the constraining means 15 can be realised in different ways as compared to that described above, provided that the tension element is able to rotate with respect to the foundation around at least one axis and within a certain angle range.

The structural roofing system 1 according to the present invention can comprise tensioning means 16 which can be used to control the length and level of tension in the tension elements 5. In other words, the tension element 5 is extended between the at least two points 30, 31 of the foundation 3 and, at later stage, tensioned by changing its length between the two points 30, 31.

The tensioning means 16 used to change the level of tension in the tension element are represented only in a schematic way in the lateral view of figure 3 and can comprise threaded turnbuckles or other devices controlled manually or by means of hydraulic actuators, which can change the level of tension in the tension element by reducing its length between the points 30, 31 between which it spans.

Turning now to the pneumatic element 10 of the roofing system 1, as already mentioned above, it is interposed between the at least one tension element 5, on which it is supported, and the covering layer 2.

In particular, the pneumatic element 10, when it is at least partially inflated, separates the covering layer from the tension element, which in turn supports the pneumatic element 10. In other words, the pneumatic element is arranged between the tension element 5 and at least part of the covering layer 2.

Advantageously, the presence of the pneumatic element 10 between the covering layer 2 and the tension element 5 allows the absorption of vibrations and movements of the covering layer and the distribution of the forces applied to the pneumatic element, so that the applied forces, instead of being concentrated in small areas, are efficaciously distributed along the entire length of the tension element 5.

Moreover, according to and advantageous aspect of the invention, in the embodiment of the invention in which the covering layer is flexible, the level of inflation of the pneumatic element, and thus the distance between the at least part of the covering layer and the tension element, allows the control of the shape assumed by the said covering layer. More specifically, according to a preferred embodiment of the invention, the presence of the pneumatic element allows to support the covering layer and, at the same time, the control of the level of tension of said covering layer in order to avoid the risk of water ponding.

Advantageously, according to the level of inflation of the pneumatic element 10 it is possible to control the distance between the covering layer, and in the case the covering layer is flexible, it is possible to control the shape and thus the level of tension of said covering layer.

The pneumatic element 10 comprises a body intended to retain at least one gas under pressure, preferably air. However, other gases can also be used such as a mixture of carbon dioxide when the structural roofing system 1 is used in application where it is not possible to use of inflammable gases.

In addition, the structural roofing system 1 comprises means for varying the size of the pneumatic element 10, i.e. means able to control the amount of gas inside the said pneumatic element. These means, not shown in the attached figures, can comprise blowers able to supply the gas into the body to the pneumatic element 10 according to a controlling system which monitors the difference between the required and the real pressure due to leakages or changes in the temperature and in the applied loads.

The body of the pneumatic element can be manufactured with several materials such as a thin layer of impermeable material designed to retain the gas inside the said pneumatic element.

According to a possible embodiment of the invention, as shown in the attached figures, the pneumatic element 10 has an elongated shape in the direction X of extension of the tension element 5.

In other words, the longitudinal axis Z of the pneumatic element 10 is extending substantially parallel to the direction X of extension of the tension element 5, in other words substantially parallel to the straight line which connects the two points 30, 31 of the foundation 3 between which the tension element is extending. According to an aspect of the invention, the pneumatic element 10 has a rectilinear longitudinal axis Z, as shown in the attached figures.

Preferably, the pneumatic element 10 has the shape of an elongated linear element designed to perform as a structural beam.

Clearly, it should be noticed that with the expression "longitudinal axis Z of the pneumatic element 10 is extending substantially parallel to the direction X of extension of the tension element 5", herein adopted, also include the embodiment wherein the longitudinal axis Z corresponds to the direction X of extension of the tension element 5.

According to a possible embodiment of the invention, as shown in the attached figures, the pneumatic element 10 lies on the tension element 5, preferably for its entire length.

As described above and shown in the attached figures, the pneumatic element 10 has at least one substantially circular cross section 10a (see the detailed view in figure 4) and the tension element 5 is arranged underneath the horizontal diameter 10b of the circular cross section 10a in order to support the pneumatic element 5. The tension element 5 supports the pneumatic element 10 from underneath, with at least a portion of the pneumatic element lying on the tension element in such a way that the pneumatic element can be supported by the tension element.

Preferably, the tension element 5 is arranged in correspondence of the surface 10e of the pneumatic element oriented towards the ground. In more detail, in the embodiment of the invention shown in the transversal section of the pneumatic element in figure 4, the two tension elements, and in particular the cables 5, support from below the pneumatic element 10, being arranged underneath the horizontal diameter 10b of the corresponding circular cross section 10a.

In addition, as shown in figure 4, the two cables 5 are arranged in a symmetric way with respect to the vertical diameter, or the vertical axis 10c of the section. In this way, it is possible to advantageously support the pneumatic element 10 avoiding any possible lateral movement.

According to a possible embodiment of the invention, not represented in the figures, means for connecting the pneumatic element 10 to the tension element 5 can be provided.

For example, the at least one tension element 5 can be inserted in a corresponding housing, pocket or rings which can be integrated into the external surface of the pneumatic element in order to firmly connect the pneumatic element 10 and the tension element 5.

In general, the tension element 5 can support the pneumatic element 10 in such a way that through its inflation it is possible to control the distance between the upper portion 10d of said pneumatic element and the tension element 5, in order to support the covering layer placed above the pneumatic element.

In particular, the covering layer 2 is at least in part placed above the pneumatic element of the structural roofing system 1 according to the invention. In this way the covering layer can be supported and the applied loads can be efficaciously transferred from the pneumatic element 10 to the tension element 5 and, finally, to the foundations 3.

It should be noted that expression covering layer 2 is used to indicate elements known in the building industry suitable for the realization of roofing arrangements such as a wide range of roofing panels and other flat or corrugated elements which are arranged above the at least one pneumatic element 10. The pneumatic element 10 keeps them at a distance from the tension element 5 in such a way that the tension element can support said panels in an indirect way by placing the at least one pneumatic element in between.

In addition, according to a preferred aspect of the present invention the covering layer is flexible. This expression means that the covering layer 2 can be folded and, more generally, deformed in order to fit the support provided by the pneumatic element 10.

The flexible covering layer can be obtained through the assembly, by gluing, welding or sewing, of portions of flat surfaces obtained from sheets or rolls of flexible materials according to specific cutting patterns.

In more detail, the flexible covering layer 2 can comprise at least one cloth realised with a flexible material such as a plastic material, or a covering layer obtained from several rigid surfaces connected to be movable one with respect to another. For example, it is possible a solution wherein rigid portions which are able to rotate, for example with a joint designed to allows the mutual rotation of the portions, for example by using one or more hinges. The said solution provide a covering layer with a flexible behaviour which is able to modify its shape through the at least one pneumatic element that is at least partially arranged underneath the said covering layer.

According to a possible embodiment of the invention, the covering layer 2 can comprise a reinforcing element such as a set of cables, ropes or belts, or a net obtained from said cables, ropes or belts and designed to support the cloth or the rigid panels forming the covering layer.

According to a preferred embodiment of the invention, the covering layer 2 comprises at least one cloth realised with a flexible material, for example a cloth realised with a plastic material, preferably an impermeable plastic material.

Advantageously, as introduced above, the covering layer is at least partially arranged above the at least one pneumatic element 10 which allows to keep the covering layer at distance from the tension elements 5, in addition the inflation of the pneumatic elements 10 allows the control of the shape assumed by the flexible cloth.

In more detail, the shape of the covering layer is controlled through the pneumatic element and, in particular, by changing the level of tension in the covering layer in order to avoid deflections or local deformations which reduce the volume underneath the covering layer and lead to water ponding above the covering layer.

In more detail, as it should be clear at this stage of the description, the modification of the distance of the upper portion 10d of the at least one pneumatic element 10 from the at least one tension element 5 allows the tensioning of the flexible covering layer. In addition, through the pneumatic element it is possible to control the curvature of the covering layer 2, preferably in order to obtain at least one portion of the covering layer with a concavity oriented downwards and, thus, towards the area or the structure which has to be covered.

Preferably, the covering layer 2 is anchored to at least one fixed point and/or directly to the ground 4, in such a way that the inflation of the at least one pneumatic element 10, arranged underneath the said covering layer, allows the increase of the volume underneath and, thus, the control of the shape assumed by the covering layer according to the desired way. In particular, in this way the covering layer can be tensioned in a rapid and simple way. The term fixed point indicates at least one anchorage point for the covering layer which, preferably, does not move when the pneumatic element 10 is inflated in order to tension the covering layer. The at least one fixed point for the anchorage of the covering layer can be, for example, integrated into the structure to be covered or into the foundation 3, or directly into the ground 4.

According to a possible embodiment of the invention the covering layer 2, and in particular the flexible cloth, can be connected to the ground (or to at least one fixed point) through metallic rings, or similar anchoring means, which, as said above, are designed to cooperate with adequate fixed points designed and installed according to the building requirements.

According to a possible embodiment of the invention, the length of the anchoring means of the covering layer can be changed in order to allow the further adjustment of the level of tension into the covering layer.

According to a possible embodiment of the invention the anchoring means of the covering layer to the ground and/or to a fixed point are arranged substantially in correspondence of the boundary of the covering layer 2, preferably with a predetermined distance between them.

According to an aspect of the present invention, even if not represented in the attached figures, the covering layer 2 is longer than the pneumatic element 10 in the direction X of extension of the tension element 5 between the at least two points 30, 31 of the foundation 3. In other words, the covering layer 2 has a length greater than the length of the pneumatic element 10, along the longitudinal axis Z, in such a way that the said covering layer can extend beyond the extremities of the pneumatic element.

In this way, it is possible to optimise the anchoring of the covering layer 2 to the ground 4 and/or to at least one fixed point. Obviously, as for example shown in figure 1, the covering layer 2 can extend laterally as compared to the at least one pneumatic element 10, and hence according to a direction perpendicular to the longitudinal axis Z, in order to allow the efficacious cover of the area underneath 50 and improving the anchoring of the covering layer 2 to the ground 4 and/or to at least one fixed point.

It should be noted that, for reasons of representation, the anchoring means of the covering layer to the ground and/or to a fixed point are not shown in the attached figures.

The present invention also relates to a method for the construction of roofing arrangements through the at least one structural roofing system 1 described above. The method comprises the step of providing of at least one foundation 3 which, as said above, is suitable to transmit the forces of the structural system to the ground, the step of constraining to the at least two points of the at least one foundation 3 at least one tension element 5, the step of supporting the at least one pneumatic element 10 through at least one tension element 5.

According to an aspect of the method, the at least one tension element 5 supports the pneumatic element 10 and at least one portion of the pneumatic element lies on said tension element.

In this way, the pneumatic element 10 and the covering layer 2 are at certain distance from the ground 4 and/or from the area underneath 50 covered by the structural system 1.

According to an aspect of the present invention, the step of supporting the pneumatic element trough the tension element can require that the pneumatic element 10 is arranged with the longitudinal axis Z oriented substantially parallel to the straight line which connects the at least two points 30, 31 of the foundation 3 between which the tension element extends.

In more detail, as said with reference to the structural roofing system 1, a pneumatic element 10 is arranged above the at least one tension element 5 in order to support the said pneumatic element. According to an aspect of the method according to the invention, this step requires that the tension element 5 is constrained between two points 30, 31 of at least one foundation 3 and suspended above the ground 4 and/or the area underneath 50 covered by the structural system 1.

The method further comprises the step of placing the covering layer 2 with at least a portion above one of the pneumatic elements 10 in such a way that the said covering layer 2 is supported by the pneumatic element. This step is carried out using known means able to supply at least one gas into the body of the pneumatic element. The means for varying the size of the pneumatic element 10 can be used in a continuous way in presence of a pneumatic element which is not completely airtight. Otherwise, the said means can be used to maintain constant the level of pressure inside the pneumatic element in case of potential leakages or variations due to different environmental conditions.

In addition, the method comprises the step of inflating the pneumatic element in order to keep at distance the covering layer from the tension element through the pneumatic element 10, which is arranged in between.

It should be noted that the steps of the method described above do not need to be carried out in the order in which they are described.

In particular, it should be noticed that the step of inflating the pneumatic element 10 can be carried out before the arrangement of the covering layer 2 above the said pneumatic element, or before the placement of the pneumatic element above the tension element.

Moreover, according to a possible embodiment of the invention, the covering layer can be arranged above the tension elements 5 and subsequently the pneumatic element, preferably deflated, is inserted in between and then inflated.

Despite this, according to a preferred embodiment of the invention, the step of inflating the pneumatic element 10 is carried out when the pneumatic element is supported by the tension element 5, with at least one portion arranged above the tension element and after that the covering layer 2 is placed, with at least a portion of said covering layer, above the pneumatic element 10.

In this way it is possible to simplify the construction of the covering, in fact, the pneumatic element can be transported in the deflated configuration and easily placed on the tension element 5, which is constrained to the foundation 3. Subsequently, the covering layer 2 is arranged above the at least one pneumatic element 10 which is inflated in order to control the distance of the covering layer from the tension element 5, so as to support the covering layer 2.

In addition, as said above, according to a preferred embodiment of the invention where the covering layer is flexible, the inflation of the pneumatic element 10 allows the control of the shape assumed by the covering layer and the level of tension applied to the said covering layer.

In fact, the method according to the invention comprises the step of anchoring the covering layer to at least one fixed point, or to the ground 4. Preferably, this step is carried out before the inflation of the pneumatic element 10 in such a way that through the successive inflation of the at least one pneumatic element, it is possible to control the distance between the covering layer 2 and the tension element 5 in order to control the shape of the flexible covering layer.

In fact, the flexible covering layer is anchored to at least one fixed point and is advantageously tensioned through the pneumatic elements 10 which are inflated underneath the covering layer.

Obviously, the size of the at least one pneumatic element 10, and thus the level of inflation, allows the advantageous control of the shape assumed by the flexible covering layer and the level of tension applied.

The structural roofing system 1 according to the present invention can be subjected to some changes without departing from the scope of the present invention.

For example, the number of pneumatic elements, tension elements and the foundations to which they are constrained, can be modified according to the size of the structure that has to be covered.

In particular, even if in the prospective view of figure 1 three pneumatic elements 1 are shown, this number should not be assumed as a limitation of the possible uses.

In addition, according to a possible embodiment of the invention, the covering layer can be provided with specific heating devices able to prevent the accumulation of snow or ice on the said covering layer in certain environmental conditions.

In order to reduce the level of mechanical stress applied to the covering layer and the tension elements, a possible embodiment of the invention can include the inflation of the entire volume enclosed by the structural roofing structure in order to counter balance at least in part the loads applied to the said structural roofing structure.

## Claims

1. Structural roofing system (1) for covering an underneath area (50) comprising at least one foundation (3) to transmit the loads of said structural roofing system to the ground (4), at least one tension element (5) extending between at least two points (30, 31) of said at least one foundation (3), at least one pneumatic element (10) supported by said at least one tension element (5) with at least one portion of said pneumatic element lying on said at least one tension element (5), and at least one covering layer (2) having at least a portion located above said at least one pneumatic element (10) and supported by said at least one pneumatic element (10), wherein said at least one pneumatic element (10) keeps at distance at least part of said covering layer (2) from said at least one tension element (5), when said at least one pneumatic element is at least partially inflated, **characterized in that** the at least one pneumatic element (10) is provided with a longitudinal axis (Z) extending substantially parallel to the straight line which connects said at least two points (30, 31) of said at least one foundation (3) between which said tension element (5) extends.

2. Structural roofing system according to claim 1, wherein said at least one pneumatic element (10) is provided with an elongated shape in the direction of extension (X) of said at least one tension element (5) between said at least two points (30, 31) of said at least one foundation (3).

3. Structural roofing system according to any previous claim, wherein said tension element (5) is constrained between at least two points (30, 31) of said at least one foundation (3) suspended with respect to the ground (4) and/or with respect to the underneath area (50) covered by the structural roofing system (1).

4. Structural roofing system according to any previous claim, wherein said at least one tension element (5) comprises at least one cable or at least one bar, extending between at least two points of said at least one foundation (3).

5. Structural roofing system according to any previous claim, wherein said at least one tension element (5) comprises at least two cables or at least two bars, extending parallel to each other between at least two points (30, 31) of said at least one foundation (3).

6. Structural roofing system according to any previous claim, wherein said at least one tension element (5) is constrained, directly or in directly, to said at least one foundation (3) by constraining means (15) allowing the rotation of said at least one tension element (5) with respect to said at least one foundation (3).

7. Structural roofing system according to any previous claim, wherein said at least one pneumatic element (10) comprises at least one substantially circular cross section (10a) and said at least one tension element (5) is located below said horizontal diameter (10b) of said circular cross section (10a) for supporting said at least one pneumatic element (5).

8. Structural roofing system according to any previous claim, wherein said covering layer (2) is flexible, said at least one pneumatic element (10) modifying at least partially the shape of said flexible covering layer, when said at least one pneumatic element is at least in part inflated.

9. Structural roofing system according to any previous claim, wherein said covering layer (2) comprises at least one cloth of flexible material.

10. Structural roofing system according to claim 8 or 9, wherein said flexible covering layer (2) is anchored to at least one fixed point and/or to the ground (4).

11. Structural roofing system according to any previous claim, **characterized in** comprising means for varying the size of said at least one pneumatic element (10) by changing the amount of gas present therein.

12. Method for the construction of roofing arrangements by at least a structural roofing system (1) according to any claim 1 to 11, comprising the steps of:
• providing at least one foundation (3) to transmit loads of said structural roofing system to the ground (4);
• constraining at least one tension element (5) between at least two points (30, 31) of said at least one foundation (3);
• supporting said at least one pneumatic element (10) by said at least one tension element (5) with at least one portion of said pneumatic element lying on said at least one tension element (5);
• placing said covering layer (2) with at least a portion thereof above said at least one pneumatic element (10);
• inflating said at least one pneumatic element (10) for spacing said covering layer (2) from said at least one tension element (5) by means of said at least one pneumatic element (10).

13. Method according to claim 12, wherein the step of inflating said at least one pneumatic element (10) is performed when said at least one pneumatic element (10) is supported by said at least one tension element (5) and after that said covering layer (2) is arranged with at least a portion above the at least one pneumatic element (10).

14. Method according to claim 12 or 13, comprising the step of anchoring to at least one fixed point and/or to the ground (4), said covering layer before performing the step of inflating said at least one pneumatic element (10).

## Patentansprüche

1. Strukturelles Dachsystem (1) zum Abdecken eines darunter liegenden Bereichs (50) umfassend wenigstens ein Fundament (3) zum Übertragen der Last des strukturellen Dachsystems in den Boden (4), wenigstens ein sich zwischen wenigstens zwei Punkten (30, 31) von dem wenigstens einen Fundament (3) erstreckendes Spannelement (5), wenigstens ein durch das wenigstens eine Spannelement (5) unterstütztes Luftelement (10), wobei wenigstens ein Teil des Luftelements auf dem wenigstens einen Spannelement (5) liegt, und wenigstens einer Abdeckschicht (2) mit wenigstens einem Teil angeordnet über dem wenigstens einen Luftelement (10) und unterstützt durch das wenigstens eine Luftelement (10), wobei das wenigstens eine Luftelement (10) wenigstens einen Teil der Abdeckschicht (2) auf Abstand von dem wenigstens einen Spannelement (5) hält, wenn das wenigstens eine Luftelement wenigstens teilweise aufgeblasen ist, **dadurch gekennzeichnet, dass** das wenigstens eine Luftelement (10) eine Längsachse (Z) aufweist, die sich im Wesentlichen parallel zu der geraden Linie erstreckt, welche die wenigstens zwei Punkte (30, 31) von dem wenigstens einen Fundament (3) verbindet zwischen denen sich das Spannelement (5) erstreckt.

2. Strukturelles Dachsystem nach Anspruch 1, wobei das wenigstens eine Luftelement (10) mit einer länglichen Form in der Richtung der Erstreckung (X) von dem wenigstens einen Spannelement (5) zwischen den wenigstens zwei Punkten (30, 31) von dem wenigstens einen Fundament (3) versehen ist.

3. Strukturelles Dachsystem nach einem beliebigen vorherigen Anspruch, wobei das Spannelement (5) gehalten wird zwischen den wenigstens zwei Punkten (30, 31) von dem wenigstens einen Fundament (3) schwebend in Bezug auf den Boden (4) und/oder in Bezug auf den darunter liegenden Bereich (50) abgedeckt durch das strukturelle Dachsystem (1).

4. Strukturelles Dachsystem nach einem beliebigen vorherigen Anspruch, wobei das wenigstens eine Spannelement (5) wenigstens ein Kabel oder wenigstens eine Stange umfasst, erstreckend zwischen wenigstens zwei Punkten von dem wenigstens einen Fundament (3).

5. Strukturelles Dachsystem nach einem beliebigen vorherigen Anspruch, wobei das wenigstens eine Spannelement (5) wenigstens zwei Kabel oder wenigstens zwei Stangen umfasst, parallel zueinander erstreckend zwischen wenigstens zwei Punkten von dem wenigstens einen Fundament (3).

6. Strukturelles Dachsystem nach einem beliebigen vorherigen Anspruch, wobei das wenigstens eine Spannelement (5) direkt oder indirekt mit dem wenigstens einen Fundament (3) verbunden ist durch Haltemittel (15), welche eine Rotation von dem wenigstens einen Spannelement (5) in Bezug auf das wenigstens eine Fundament (3) erlauben.

7. Strukturelles Dachsystem nach einem beliebigen vorherigen Anspruch, wobei das wenigstens eine Luftelement (10) einen im Wesentlichen runden Querschnitt (10a) umfasst und das wenigstens eine Spannelement (5) unterhalb des horizontalen Durchmessers von dem runden Querschnitt (10a) angeordnet ist um das wenigstens eine Luftelement (5) zu stützen.

8. Strukturelles Dachsystem nach einem beliebigen vorherigen Anspruch, wobei die Abdeckschicht (2) flexibel ist, wobei das wenigstens eine Luftelement (10) zumindest teilweise die Form der flexiblen Abdeckschicht verändert, wenn das wenigstens eine Luftelement wenigstens teilweise aufgeblasen ist.

9. Strukturelles Dachsystem nach einem beliebigen vorherigen Anspruch, wobei die Abdeckschicht (2) wenigstens ein Tuch aus flexiblem Material umfasst.

10. Strukturelles Dachsystem nach Anspruch 8 oder 9, wobei die flexible Abdeckschicht (2) an wenigstens einem festen Punkt und/oder am Boden (4) verankert ist.

11. Strukturelles Dachsystem nach einem beliebigen vorherigen Anspruch, **gekennzeichnet durch** Mittel zur Veränderung der Größe von dem wenigstens einen Luftelement (10) **durch** eine Änderung der Menge an darin befindlichem Gas.

12. Verfahren zum Aufbauen einer Dachanordnung durch wenigstens ein strukturelles Dachsystem (1) nach einem der Ansprüche 1 bis 11, umfassend die Schritte:
• bereitstellen von wenigstens einem Fundament (3) zum Übertragen der Last des strukturellen Dachsystems in den Boden (4);
• aufhängen von wenigstens einem Spannelement (5) zwischen wenigstens zwei Punkten (30, 31) von dem wenigstens einen Fundament (3);
• stützen von wenigstens einem Luftelement (10) durch das wenigstens eine Luftelement (5), wobei wenigstens ein Teil des Luftelements auf dem wenigstens einen Spannelement (5) liegt;
• platzieren der Abdeckschicht (2) mit wenigstens einem Teil davon oberhalb des wenigstens einen Luftelements (10);
• aufblasen des wenigstens einen Luftelements (10) zum Beabstanden der Abdeckschicht (2) von dem wenigstens einen Spannelement (5) durch das wenigstens eine Luftelement (10).

13. Verfahren nach Anspruch 12, wobei der Schritt des Aufblasens des wenigstens einen Luftelements (10) durchgeführt wird, wenn das wenigstens eine Luftelement (10) durch das wenigstens eine Spannelement (5) gestützt wird und nachdem die Abdeckschicht (2) mit wenigstens einem Teil oberhalb des wenigstens einen Luftelements (10) angeordnet ist.

14. Verfahren nach Anspruch 12 oder 13, umfassend den Schritt des Verankerns der Abdeckschicht an wenigstens einen festen Punkt und/oder am Boden (4) vor dem Ausführen des Schritts des Aufblasens des wenigstens einen Luftelements (10).

## Revendications

1. Système structurel de couverture de toit (1) destiné à couvrir une zone de dessous (50) comprenant au moins une fondation (3) pour transmettre les charges dudit système structurel de couverture de toit au sol (4), au moins un élément de tension (5) s'étendant entre au moins deux points (30, 31) de ladite au moins une fondation (3), au moins un élément pneumatique (10) supporté par ledit au moins un élément de tension (5) avec au moins une portion dudit élément pneumatique reposant sur ledit au moins un élément de tension (5), et au moins une couche couvrante (2) ayant au moins une portion située au-dessus dudit au moins un élément pneumatique (10) et supportée par ledit au moins élément pneumatique (10), dans lequel ledit au moins un élément pneumatique (10) garde à distance au moins une partie de ladite couche couvrante (2) dudit au moins un élément de tension (5), lorsque ledit au moins un élément pneumatique est au moins partiellement gonflé, **caractérisé en ce que** l'au moins un élément pneumatique (10) est pourvu d'un axe longitudinal (Z) s'étendant sensiblement parallèlement à la droite qui relie lesdits au moins deux points (30, 31) de ladite au moins une fondation (3) entre lesquels ledit élément de tension (5) s'étend.

2. Système structurel de couverture de toit selon la revendication 1, dans lequel ledit au moins un élément pneumatique (10) est pourvu d'une forme allongée dans la direction d'extension (X) dudit au moins un élément de tension (5) entre lesdits au moins deux points (30, 31) de ladite au moins une fondation (3).

3. Système structurel de couverture de toit selon une quelconque revendication précédente, dans lequel ledit élément de tension (5) est contraint entre au moins deux points (30, 31) de ladite au moins une fondation (3) suspendue par rapport au sol (4) et/ou par rapport à la zone de dessous (50) couverte par le système structurel de couverture de toit (1).

4. Système structurel de couverture de toit selon une quelconque revendication précédente, dans lequel ledit au moins un élément de tension (5) comprend au moins un câble ou au moins une barre, s'étendant entre au moins deux points de ladite au moins une fondation (3).

5. Système structurel de couverture de toit selon une quelconque revendication précédente, dans lequel ledit au moins un élément de tension (5) comprend au moins deux câbles ou au moins deux barres, s'étendant parallèlement l'une à l'autre entre au moins deux points (30, 31) de ladite au moins une fondation (3).

6. Système structurel de couverture de toit selon une quelconque revendication précédente, dans lequel ledit au moins un élément de tension (5) est contraint, directement ou indirectement, sur ladite au moins une fondation (3) par un moyen de contrainte (15) permettant la rotation dudit au moins un élément de tension (5) par rapport à ladite au moins une fondation (3).

7. Système structurel de couverture de toit selon une quelconque revendication précédente, dans lequel ledit au moins un élément pneumatique (10) comprend au moins une section sensiblement circulaire (10a) et ledit au moins un élément de tension (5) est situé en dessous dudit diamètre horizontal (10b) de ladite section circulaire (10a) pour supporter ledit au moins un élément pneumatique (5).

8. Système structurel de couverture de toit selon une quelconque revendication précédente, dans lequel ladite couche couvrante (2) est flexible, ledit au moins un élément pneumatique (10) modifiant au moins partiellement la forme de ladite couche couvrante flexible, lorsque ledit au moins un élément pneumatique est au moins en partie gonflé.

9. Système structurel de couverture de toit selon une quelconque revendication précédente, dans lequel ladite couche couvrante (2) comprend au moins une étoffe de matière flexible.

10. Système structurel de couverture de toit selon la revendication 8 ou 9, dans lequel ladite couche couvrante flexible (2) est ancrée sur au moins un point fixe et/ou au sol (4).

11. Système structurel de couverture de toit selon une quelconque revendication précédente, **caractérisé en ce qu'**il comprend des moyens pour faire varier la taille dudit au moins un élément pneumatique (10) en changeant la quantité de gaz présent à l'intérieur.

12. Procédé de construction d'agencements de couverture de toit par au moins un système structurel de couverture de toit (1) selon l'une quelconque des revendications 1 à 11, comprenant les étapes de :
• fourniture d'au moins une fondation (3) pour transmettre des charges dudit système structurel de couverture de toit au sol (4) ;
• contrainte d'au moins un élément de tension (5) entre au moins deux points (30, 31) de ladite au moins une fondation (3) ;
• support dudit au moins un élément pneumatique (10) par ledit au moins un élément de tension (5) avec au moins une portion dudit élément pneumatique reposant sur ledit au moins un élément de tension (5) ;
• placement de ladite couche couvrante (2) avec au moins une portion de celle-ci au-dessus dudit au moins un élément pneumatique (10) ;
• gonflage dudit au moins un élément pneumatique (10) pour espacer ladite couche couvrante (2) dudit au moins un élément de tension (5) au moyen dudit au moins un élément pneumatique (10).

13. Procédé selon la revendication 12, dans lequel l'étape de gonflage dudit au moins un élément pneumatique (10) est réalisée lorsque ledit au moins un élément pneumatique (10) est supporté par ledit au moins un élément de tension (5) et après que ladite couche couvrante (2) est agencée avec au moins une portion au-dessus de l'au moins un élément pneumatique (10).

14. Procédé selon la revendication 12 ou 13, comprenant l'étape d'ancrage sur au moins un point fixe et/ou sur le sol (4), de ladite couche couvrante avant de réaliser l'étape de gonflage dudit au moins un élément pneumatique (10).
